# EUROPEAN PATENT APPLICATION

(11) **EP 2 901 946 A1**
(43) Date of publication of application: **05.08.2015**
(21) Application number: 14153714.2
(22) Date of filing: 03.02.2014
(51) Int. Cl.: A61B 17/16, A61B 17/17, A61B 19/00

(54) **Pointing device and drilling tool**

(71) Applicant: Arthrex Inc, Naples, FL 34108-1945 (US)
(72) Inventor: Schmieding, Reinhold, Naples, Florida 34109 (US); Michel, Gerlinde, 80999 Munich (DE)
(74) Representative: Lohr, Georg

(57) **Abstract**

An arthroscopic drill guiding system for drilling a hole into a bone comprises an arthroscopic pointing device, a drill sensor and an indicator. The pointing device has a shaft with a distal section and at least one navigation sensor for locating a target position distally at the bone. The drill sensor is coupled to a drill and provides information about the position and/or direction of the drill. The indicator indicates the relative position of the drill with respect to the target position, thus allowing to align the drill with the target position.

## Description

### Field of the invention

The invention relates to the field of surgery and, more particularly, to improved instruments and methods of surgery.

### Description of the Related Art

During surgical procedures, it is often necessary to approach a specific anatomical location precisely and directly, to avoid unwanted surgical trauma and to place soft tissue or a bone drill tunnel or socket in an accurate location. These procedures need to rely on some sort of instrument position monitoring, to ensure proper access to, and behavior at, the target location.

For example, orthopedic surgery and related trauma care requires bone drilling when fracture repair is indicated. The holes formed by drilling are configured to accept fixation devices such as screws to hold implants such as bone plates or prostheses, or to exert pressure between broken bone fragments. The depth of the drilling of the bone while protecting the surrounding soft tissue, the size and lengths of the screws to be fixated, the position of the bone plate or prosthesis, all require precise monitoring of instruments and procedures (i.e., accurate insertion and placement of instruments, insertion of various fixation devices at different parameters such as depth, angle, etc., conducting various surgical steps under predetermined conditions and parameters, etc.).

A system and a method for identifying landmarks on orthopedic implants is disclosed in the US Patent Application Publication No. 2010/0152573 A1. A first magnetic sensor is mounted to the orthopedic implant while a second magnetic sensor is attached to a drill guide. This allows for alignment of the drill guide with holes in the orthopedic implant. The orthopedic implant is comparatively large to hold magnetic sensors and requires a major surgery.

### SUMMARY OF THE INVENTION

The problem to be solved by the invention is to provide a means that can be used without major surgery and which does not require the use of other implants or complex instruments. Targeting and/or using the means should not impose additional tasks or additional workload to the surgeon. Handling should be very simple.

Solutions of the problem are described in the independent claims. The dependent claims relate to further improvements of the invention.

A first embodiment relates to a universal pointing device. The pointing device has an elongated and preferably straight shaft with a proximal end and a distal section, which may be curved. The shaft preferably comprises a tube, most preferably a metal tube, which may have a tip at its distal end. The shaft may also be a wire to which the sensor is attached. Preferably, the distal end of the pointing device is used for identifying a location. The pointing device includes at least one miniature navigation sensor. Preferably, navigation sensor is a magnetic or an inductive navigation sensor. The navigation sensor may be coupled to the shaft, preferably to the end of the shaft. With a navigation sensor coupled to the end of the shaft, the navigation sensor itself can be brought to the location to be identified. Therefore, a small 3- axis navigation sensor would be sufficient to determine a position in a 3- dimensional space. Preferably, the magnetic sensor is encapsulated into the shaft. The sensor may be located outside a metal tube preferably at the end of the tube, and further embedded into plastic material, which may be a shrink tube.

In an alternative embodiment, the navigation sensor is located distant from the end of the shaft. Here, at least a 5-axis navigation sensor most preferably a 6-axis sensor, would be required to identify the position of the end of the shaft. The sensor determines the position in three-dimensional space and the direction of the shaft in the 3- dimensional space. Knowing these parameters, the position of the end of the shaft can be calculated. Alternatively, two 3 axis navigation sensors may be held in the shaft to determine the position and the orientation of the shaft in a 3- dimensional space. Having these values, again, the position of the end of the shaft can be calculated.

The pointing device may be used to point to a specific location in a patient's body, preferably to a location at a bone, which may be at the surface of the bone. This location may be the distal end point of a hole to be drilled into the bone. A target position can easily be located by touching the position with the and of the shaft or tip of the pointing device. Alternatively, the target position may have an offset of a predetermined distance in a predetermined direction from the navigation sensor and/or from the tip. An offset position can only be determined, if the position and the orientation of the shaft in 3-dimensional space have been determined.

Preferably the shaft has a diameter which allows accessing the desired location through minimal invasive holes, therefore avoiding major surgery. Therefore, the preferred diameter is less than 5 mm, most preferably less than 3 mm. It is preferred, if the shaft forms a tube for holding and guiding wires or cables which are necessary for communication with the navigation sensor. The tube may also be a cable, a rod or any profile like for example an arc shaped profile or a U-shaped profile. Preferably, the navigation sensor is a miniature navigation sensor, most preferably a 2 mm sensor.

Furthermore, it is preferred, if the shaft is flexible or at least semi-rigid which would allow to bend the tube at least once. The shaft may be adapted to any form and/or curve which is required to reach a specific position within the body, preferably at a bone or a joint. There may be a calibration necessary to compensate for modifications of the shaft. Such a calibration can be done easily before using the device by touching a reference position with the end of the shaft and reading the position and/or direction values.

Preferably the shaft comprises non ferromagnetic material, which may be titanium or any alloy thereof or Nitinol. This allows embedding of the at least one navigation sensor into the shaft, which results in a much more robust device.

The pointing device may be used alone. For this purpose, a handle may be attached to the proximal end of the device. Preferably, the pointing device is coupled to (attached to or mounted on) a variety of delivery instruments (for example, spinal malleable surgical needles, drills, drill guides, cannulas, wire probes, arthroscopes, etc.) to provide navigated drill guide systems that confer improved precision and reliability of different surgical procedures. The sensor may be easily attached to and detached from various surgical instruments and related instrumentation that allow a single surgeon the ability to operate, use instruments arthroscopically, target area of interest, and obtain a warning when instrumentation is close to the target, with minimal trauma to adjacent tissue. Furthermore, there are no additional handling steps necessary for operating the pointing device.

A drill guiding system comprises a pointing device for indicating a target position, a drill sensor which may also be a drill guide and an indicator. The pointing device preferably is used for indicating the position of the distal end of a hole to be drilled. The drill sensor comprises at least one navigation sensor for indicating the position and/or the orientation of the drill. The drill sensor may be integrated within a drill or a motor driven handpiece.

The drill guide is a tool for guiding a drill, further having at least one navigation sensor for indicating the position and/or the orientation of the drill guide. It is preferred, if the drill guide is a sleeve, most preferably a telescoping drill sleeve. It may have at least one resistance spring which cannula end pressure against the bone during single hand drilling and telescoping component bottoms out at predetermined depth set as a customized drill stop. Furthermore, it may have a sensor coil.

Preferably, at least one of the pointing device for indicating a target position, the drill sensor, and the at least one navigation sensor in the drill guide are connected to a measurement and control unit and/or display unit. The connection may be a wired connection using cables or a wireless connection using any wireless protocol like Bluetooth, WUSB, WLAN, UWB communication and others.

The indicator serves for indicating the relative position of the drill or the drill guide with respect to the target position. It may indicate the distance and/or the relative direction. It furthermore may indicate how to move the drill guide to point to at the target position and how deep further drilling must be done. Preferably the indicator is part of the display unit. Most preferably it is part of a multiple function display unit indicating the surgeon all essential parameters like images of the operation side, which may be endoscopic images and/or x-ray images. The indicator may also indicate that the end of the hole is reached. Preferably, the indicator uses a crosshair or a bull's eye for indicating the displacement and/or correct alignment. Furthermore, the indicator may indicate the drilling distance or the remaining distance. It may further act as a safety feature by turning the bull's eye target off or turning the bull's eye red on as the drill comes to within 1-2 mm of the target position. Furthermore, there may be a marker changing its colour or blinking, when the drill reaches a predetermined position or distance to the target position.

If the end of the hole is reached, the drilling process may be stopped, for example by switching off a motor rotating the drill. After this stopping, the drill may be restarted again, manually, for example by pressing a button. The end of the hole may be detected by evaluating the distance between the end of the pointing device, indicating a target position and the drill guide. In the case of a stud hole, the end of the hole is detected by reaching a predetermined distance or position. The end of a through hole may be detected if the distance falls below a predetermined limit. Another method for detecting the end of the hole would be to identify sudden position changes, which happen, when the drill moves out of the hole. Further methods of detecting, that the drill has gone through the bone are, measuring a drill motor current, which increases when the load decreases or measuring an increase of motor speed caused by decreasing load.

A further aspect of the invention relates to a method for drilling a hole into a bone by first identifying the position of the distal end of the hole by pointing with the distal end of the pointing device to said position. Second, a drill is aligned to point to a said position. Alignment may either be done by using a drill sensor or a drill guide as described above. Then the drill is rotated to drill the hole into the bone. The drill reaching the end of the bone may be detected and indicated as described above and drilling may be determined automatically.

The pointing device may be part of or mounted on a variety of delivery instruments as follows, the list below being only an exemplary list:
An arthroscope, with at least one navigation sensor close to its distal end and/or at least one navigation sensor in its sheath and/or at least one navigation sensor close to its proximal end. There may be a special pointing arthroscope with a pointer at its distal end. The pointer may be a tube or rod or wire shaped structure which may be formed or bent to have a distal end visible through the arthroscope and which may be used to point to the desired location.

A malleable spinal needle with a navigation sensor of the present invention at the tip. For example, a SU 12 gauge (or smaller) malleable spinal needle is provided with a navigation sensor at the tip. This system may be employed for fracture targeted drilling (control of needle placement). The needle may be placed under X ray or ultrasound control to the back of a fracture site. Targeted drilling may be performed as described above. Due to the drill depth measurement, through holes or holes with a predetermined depth may be made for insertion of screws, knotless tightrope, BC dowels or elastic bio bone cement into drill holes.

This same needle could be used with a shoehorn cannula clip to an arthroscope sheath. The clip (for example, snap clip) has a pathway to insert the needle through the clip parallel to the sheath until the tip is placed in the center of a 30 degree scope image and locked. The Sensor needle can be inserted after the scope is placed in joint, to eliminate soft tissue impingement. This creates a combined optical image and drill target sensor in one.

This same needle could further be used with a drill sleeve shoehorn clip to reduce cost of disposable sensors by combining two 12 gauge sensor needles with a connecting wire. This option affords the lowest manufacturing costs and increased versatility.

In parallel, a sensor needle and a 12 gauge needle may be combined for small diameter Fiberscope and sensor needle for percutaneous, image guided targeting of needle placement.

The instruments, systems and methods may be applied to a variety of fields, for example, sports medicine, DEX, Trauma, etc.

### Brief Description of the Drawings

In the following the invention will be described by way of example, without limitation of the general inventive concept, on examples of embodiment with reference to the drawings.
Figure 1 shows an arthroscope with a pointing device.
Figure 2 shows an arthroscope with an insertion cannula.
Figure 3 a view through the arthroscope.
Figure 4 illustrates a drill guiding system.
Figure 5 illustrates a drill with integrated navigation sensor.
Figure 6 illustrates an exemplary display.
Figure 7 illustrates calibration of a pointing device.

### DETAILED DESCRIPTION OF THE PREFERRED EMBODIMENTS

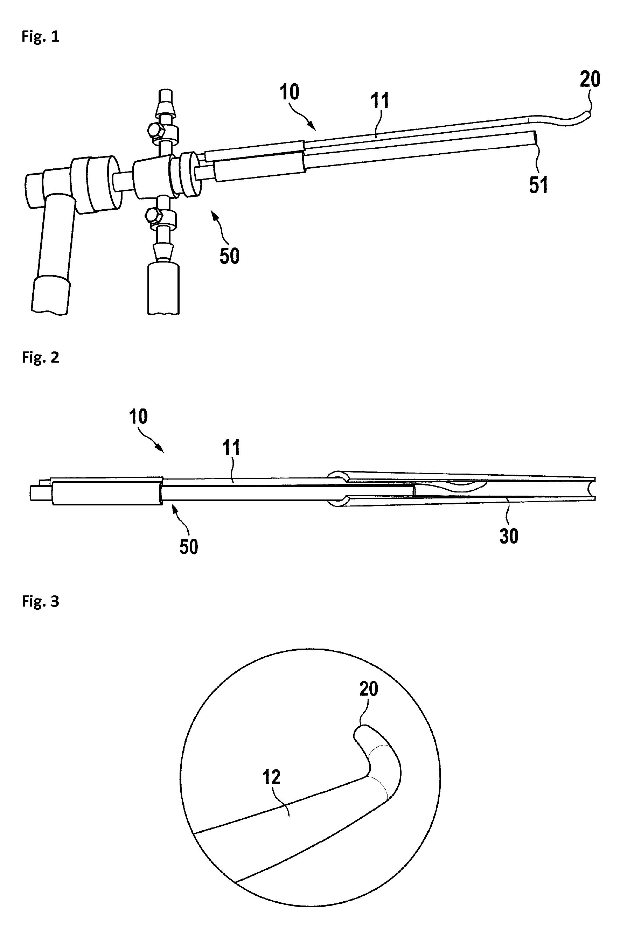

FIG. 1 shows an arthroscope 50 with a pointing device 10. The pointing device comprises a tube forming a straight shaft 11 and a curved distal section 12 with a navigation sensor 20 at its end. The straight shaft 11 is guided parallel to the arthroscope and can easily be inserted through an opening into the body. The curved distal section of the pointing device is close to the distal end 51 of the arthroscope, such that it's end with the navigation sensor 20 is visible through the arthroscope.

FIG. 2 illustrates the system of FIG. 1 with an insertion cannula 30. Due to the smaller diameter of the pointing device, only minimal additional space is required. Therefore, the pointing device may be inserted together with an arthroscope into a standard insertion cannula.

FIG. 3 shows a view through the arthroscope 50 of the previous figures. It shows the curved section of the pointing device 10. The navigation sensor 20 at the distal end of the pointing device can not to be seen here, as it is fully embedded into the tube of the pointing device.

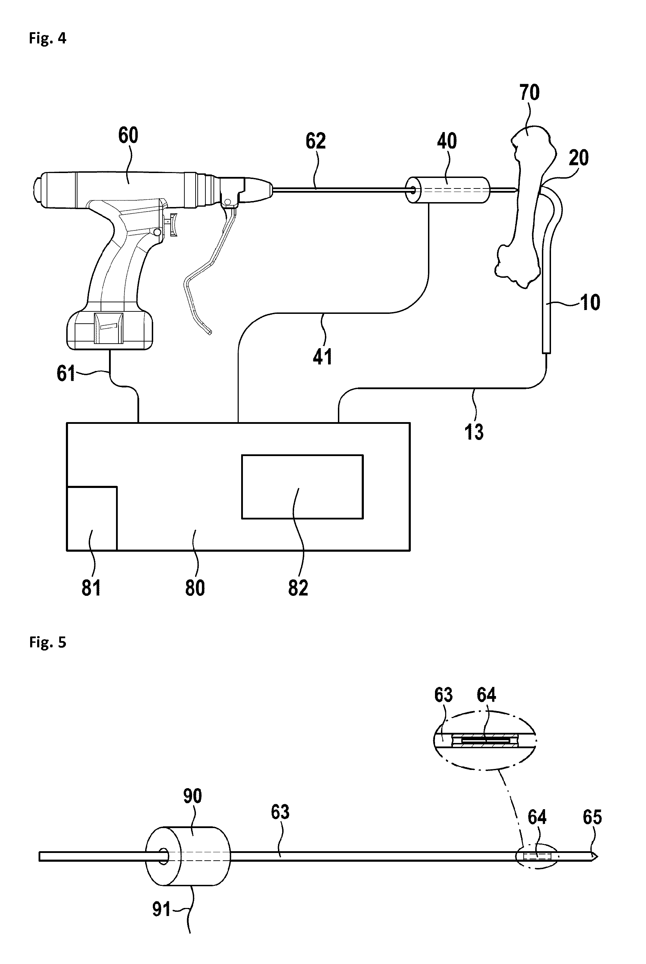

FIG. 4 illustrates a drill guiding system. A drill 62 is held and rotated by a handpiece 60. The handpiece may comprise a motor and a gear for driving the drill. The drill is guided by a drill guide 40. The drill guide preferably has the form of a sleeve with an inner bore for guiding the drill 62. The drill guide further has at least one navigation sensor for indicating the position and/or the orientation of the drill guide. The drill drills into a first side of a bone 70 starting with a proximal end of a hole. A pointing device 10 having a navigation sensor 20 is located at an opposing side of the bone, indicating the distal position of the hole to be drilled. The navigation sensor 20 and the drill guide 40 are connected via cables 13, 41 to a measurement and control unit 81 for acquiring position data and a display unit 82 for displaying the positions, which may be part of multifunction display 80. All theses units may also be in separate housings. Instead of the cables, a wireless communication may be used. The hand piece may be connected by a cable 61 to the a measurement and control unit 81 for transmitting data of an integrated navigation sensor from the handpiece and/or for transmitting control signals, like a motor stop signal to the handpiece.

FIG. 5 illustrates a drill with integrated navigation sensor 64. In this embodiment the navigation sensor is close to the distal tip 65 of the drill 63. A slip-ring 90 also called rotary joint is used to connect the rotating drill via cable 91 to the measurement and control unit 81. There may be a second navigation sensor included into the slip-ring housing.

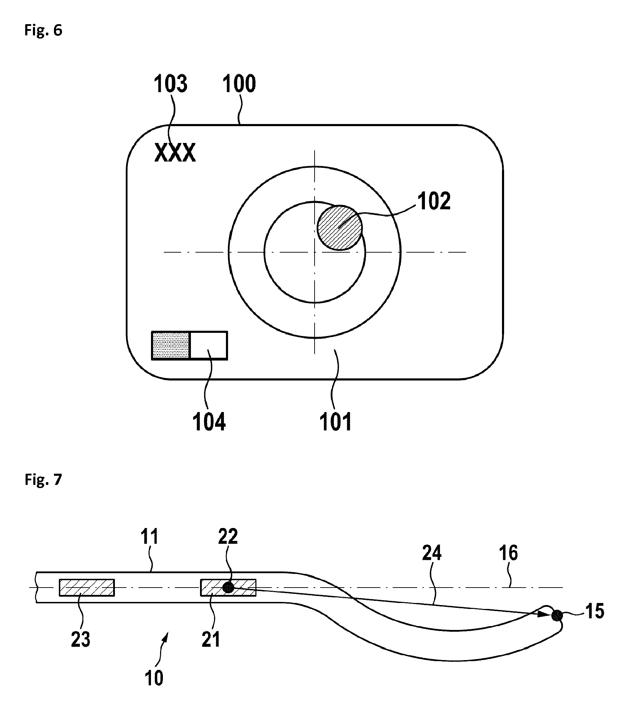

FIG. 6 illustrates an exemplary display 100 on display unit 82. There may be cross hairs 101 or bull's eye to simplify targeting. A drill position marker 102 indicates the position of the drill 62, 63 or the drill guide 40 with respect to navigation sensor 20 which defines the center of the crosshairs. There may be further indicators like a numerical value 103 or a bar indicator 104 indicating the distance between the drill (-tip) and the navigation sensor 20. As a safety feature, the bull's eye target may be turned off or the bull's eye may be turned red as the drill comes to within a predetermined distance, which may be 1-2 mm of the target position.

FIG. 7 shows calibration of a pointing device. The pointing device 10 includes at least a first navigation sensor 21. This sensor is not necessarily at or close to the distal end 15 of the pointing device, although this would be the preferred location. Instead, it is generally preferred to place the sensor at a section of the pointing device which is comparatively rigid and will not be bent, thus avoiding damaging of the sensor. Preferably, the sensor is at least a 5 axis sensor, most preferably a 6-axis sensor, providing information about its position and about its orientation in three dimensional space. By calculation, measuring or calibration, the vector 24 between the reference point 22 of the sensor 21 and the distal end 15 of the pointing device can be determined. In most cases this vector has a length and a direction, the direction being different from the center axis 16 of the shaft 11 of the pointing device. If the direction is the same, which means that the distal end 15 of the pointing device is on the center axis 16 of the shaft, then a rotation of the pointing device can be ignored and a 5- axis sensor would be sufficient. A further navigation sensor 23 may be located at a different position of the shaft 11. This sensor may serve to increase accuracy. Furthermore, there may be two lower cost 3-axis sensors 21, 23 which together deliver the required information about the position and orientation of the pointing device.

The systems and methods of the present invention allow a single surgeon the ability to operate, use instruments arthroscopically, target an area of interest, and obtain a warning when instrumentation is too close to the target, all which could be conducted by employing instruments with the navigation sensor of the present invention.

## Claims

1. An arthroscopic drill guiding system for drilling a hole into a bone comprising an arthroscopic pointing device, a drill sensor and an indicator, wherein
- the pointing device comprises a shaft with a distal section and at least one navigation sensor for locating a target position distally at the bone,
- the drill sensor is coupled to a drill and provides information about the position and/or direction of the drill, and
- the indicator indicates the relative position of the drill with respect to the target position.

2. The drill guiding system according to claim 1, wherein the drill sensor is a navigation sensor within the drill.

3. The drill guiding system according to claim 2, wherein a slip-ring is provided co electrically connect the navigation sensor within the drill.

4. The drill guiding system according to claim 1, wherein the drill sensor is a navigation sensor within a drill guide forming a sleeve to guide the drill.

5. The drill guiding system according to claim 1, wherein the navigation sensors are connected to a measurement and control unit and to a display unit, the display unit showing the indicator.

6. The drill guiding system according to claim 1, wherein the indicator comprises crosshair or a bull's eye further showing a drill position marker.

7. An arthroscopic pointing device for locating a target position within a human or animal body, comprising at least one navigation sensor, wherein the device further comprises a straight shaft with a curved distal section, the navigation sensor being located close to the distal end of the curved distal section.

8. The pointing device according to claim 7, wherein the navigation sensor is a 3-axis sensor providing position information in a 3-dimensional space.

9. An arthroscopic pointing device for locating a target position within a human or animal body, comprising at least one navigation sensor, wherein the device further comprises a straight shaft with a curved distal section, the navigation sensor being located within the shaft and distant from the curved distal section, wherein the navigation sensor is at least a 5-axis sensor providing position and direction information in a 3-dimensional space.

10. The pointing device of claim 9, wherein the navigation sensors are inductive or magnetic sensors.

11. The pointing device of claim 7, wherein the shaft of the pointing device comprises non- magnetic material.

12. The pointing device of claim 7, wherein the shaft of the pointing device comprises a tube.

13. The pointing device of claim 7, wherein the shaft of the pointing device has a diameter of less than 3mm.

14. The drill guiding system or pointing device according to claim 1, wherein the indicator comprises a marker changing its color and/or blinking when the drill sensor indicates the drill reaching a predetermined position and/or distance to the target position.
